# EUROPÄISCHE PATENTANMELDUNG

(11) **EP 3 385 248 A1**
(43) Veröffentlichungstag der Anmeldung: **10.10.2018**
(21) Anmeldenummer: 17165594.7
(22) Anmeldetag: 07.04.2017
(51) Int. Cl.: C07C 2/84, C07C 7/00, C07C 7/04

(54) **VERFAHREN UND ANLAGE ZUR GEWINNUNG EINES ODER MEHRERER OLEFINE**

(71) Anmelder: Linde AG, 80331 München (DE)
(72) Erfinder: FRITZ, Helmut, 81375 München (DE); ERNST, Christian, 85521 Ottobrunn (DE); SINN, Tobias, 81545 München (DE)
(74) Vertreter: Frommberger, Moritz

(57) **Zusammenfassung**

Die Erfindung betrifft ein Verfahren (100, 200) zur Gewinnung eines oder mehrerer Olefine, bei dem unter Einsatz einer oxidativen Kopplung von Methan (10) ein Wasserstoff, Methan, Kohlenmonoxid und höher als Methan siedende Kohlenwasserstoffe enthaltendes Gasgemisch gebildet und einer Tieftemperaturtrennung (1-5) unterworfen wird, dadurch gekennzeichnet, dass die Tieftemperaturtrennung (1-5) unter Verwendung einer Rektifikationskolonne (2) mit einem ersten Trennbereich (21), einem oberhalb des ersten Trennbereichs (21) angeordneten zweiten Trennbereich (22) und einem Kondensatorverdampfer (23) durchgeführt wird, wobei das Gasgemisch abgekühlt, zumindest teilweise als erster Trenneinsatz in den ersten Trennbereich (21) eingespeist und in dem ersten Trennbereich (21) unter Bildung eines ersten Kopfgases und einer ersten Sumpfflüssigkeit einer ersten Rektifikation unterworfen wird, wobei unter Verwendung eines ersten Anteils des ersten Kopfgases in dem Kondensatorverdampfer (23) ein Kondensat, das auf den ersten Trennbereich zurückgeführt wird, und unter Verwendung eines zweiten Anteils des Kopfgases ein zweiter Trenneinsatz, der in den zweiten Trennbereich (22) eingespeist wird, gebildet werden, und wobei der zweite Trenneinsatz in dem zweiten Trennbereich unter Bildung eines zweiten Kopfgases und einer zweiten Sumpfflüssigkeit einer zweiten Rektifikation unterworfen wird.

## Beschreibung

Die Erfindung betrifft ein Verfahren zur Gewinnung eines oder mehrerer Olefine und eine entsprechende Anlage gemäß den Oberbegriffen der jeweiligen unabhängigen Patentansprüche.

### Stand der Technik

Die oxidative Kopplung von Methan ist in der Literatur beschrieben, beispielsweise bei J.D. Idol et al., "Natural Gas", in: J.A. Kent (Hrsg.), "Handbook of Industrial Chemistry and Biotechnology", Band 2, 12. Auflage, Springer, New York 2012.

Die oxidative Kopplung von Methan umfasst nach derzeitigem Kenntnisstand eine katalysierte Gasphasenreaktion von Methan mit Sauerstoff, bei der von zwei Methanmolekülen jeweils ein Wasserstoffatom abgespalten wird. Die dabei entstehenden Methylradikale reagieren zunächst zu einem Ethanmolekül. Bei der Reaktion wird ferner ein Wassermolekül gebildet. Bei geeigneten Verhältnissen von Methan zu Sauerstoff, geeigneten Reaktionstemperaturen und der Wahl geeigneter Katalysebedingungen erfolgt anschließend eine Oxydehydrierung des Ethans zu Ethylen, einer Zielverbindung bei der oxidativen Kopplung von Methan. Hierbei wird ein weiteres Wassermolekül gebildet. Der eingesetzte Sauerstoff wird bei den genannten Reaktionen typischerweise vollständig umgesetzt.

Die Reaktionsbedingungen bei der oxidativen Kopplung von Methan umfassen klassischerweise eine Temperatur von 500 bis 900 °C, einen Druck von 5 bis 10 bar und hohe Raumgeschwindigkeiten. Jüngere Entwicklungen gehen insbesondere auch in Richtung der Verwendung tieferer Temperaturen. Die Reaktion kann homogen- und heterogenkatalytisch im Festbett oder in der Wirbelschicht erfolgen. Bei der oxidativen Kopplung von Methan können auch höhere Kohlenwasserstoffe mit bis zu sechs oder acht Kohlenstoffatomen gebildet werden, der Schwerpunkt liegt jedoch auf Ethan bzw. Ethylen und ggf. noch Propan bzw. Propylen.

Insbesondere aufgrund der hohen Bindungsenergie zwischen Kohlenstoff und Wasserstoff im Methanmolekül sind die Ausbeuten bei der oxidativen Kopplung von Methan vergleichsweise gering. Typischerweise werden nicht mehr als 10 bis 15% des eingesetzten Methans umgesetzt. Außerdem begünstigen die vergleichsweise harschen Reaktionsbedingungen und Temperaturen, die zur Spaltung dieser Bindungen erforderlich sind, auch die weitere Oxidation der Methylradikale und anderer Intermediate zu Kohlenmonoxid und Kohlendioxid.

Wenngleich den geringen Ausbeuten und der Bildung von Kohlenmonoxid und Kohlendioxid teilweise durch die Wahl optimierter Katalysatoren und angepasster Reaktionsbedingungen entgegengewirkt werden kann, enthält ein bei der oxidativen Kopplung von Methan gebildetes Gasgemisch neben den Zielverbindungen wie Ethylen und ggf. Propylen überwiegend nicht umgesetztes Methan sowie Kohlendioxid, Kohlenmonoxid und Wasser. Durch ggf. erfolgende nichtkatalytische Spaltreaktionen können auch beträchtliche Mengen an Wasserstoff enthalten sein. Ein derartiges Gasgemisch wird im hier verwendeten Sprachgebrauch auch als "Produktgemisch" der oxidativen Kopplung von Methan bezeichnet, obwohl es überwiegend nicht die gewünschten Produkte, sondern auch das nicht umgesetzte Edukt Methan und die soeben erläuterten Nebenprodukte enthält.

Bei der oxidativen Kopplung von Methan können Reaktoren eingesetzt werden, in denen einer katalytischen Zone eine nichtkatalytische Zone nachgeschaltet ist. Das aus der katalytischen Zone ausströmende Gasgemisch wird in die nichtkatalytische Zone überführt, wo es zunächst noch auf den vergleichsweise hohen Temperaturen vorliegt, die in der katalytischen Zone eingesetzt werden. Insbesondere durch die Anwesenheit des bei der oxidativen Kopplung von Methan gebildeten Wassers ähneln die Reaktionsbedingungen hier jenen herkömmlicher Dampfspaltverfahren (engl. Steam Cracking). Daher können hier Ethan und höhere Paraffine zu Olefinen umgesetzt werden. In die nichtkatalytische Zone können auch weitere Paraffine eingespeist werden, so dass die Restwärme der oxidativen Kopplung von Methan in besonders vorteilhafter Weise ausgenutzt werden kann. Ein derartiges gezieltes Dampfspalten in einer der katalytischen Zone nachgeschalteten nichtkatalytischen Zone wird auch als "Post Bed Cracking" bezeichnet. Nachfolgend wird hierfür auch der Begriff "postkatalytisches Dampfspalten" verwendet.

Bezüglich den beim Dampfspalten herrschenden Reaktionsbedingungen sei ebenfalls auf Fachliteratur wie den Artikel "Ethylene" in Ullmann's Encyclopedia of Industrial Chemistry, Onlineausgabe, 15. April 2007, DOI 10.1002/14356007.a10_045.pub2, verwiesen. Das Dampfspalten wird beispielsweise zur Gewinnung von kurzkettigen Olefinen wie Ethylen und Propylen, Diolefinen wie Butadien oder von Aromaten eingesetzt, ist jedoch nicht hierauf beschränkt.

Produktgemische, die unter Verwendung eines Verfahrens zur oxidativen Kopplung von Methan gebildet werden, enthalten aus den oben erläuterten Gründen insbesondere große Mengen an Methan, das vorteilhafterweise abgetrennt und in die oxidative Kopplung zurückgeführt werden sollte. Die trenntechnische Bearbeitung eines entsprechenden Produktgemischs stellt sich dabei aufgrund des hohen Methananteils bei dem vergleichsweise geringem Gehalt an Zielprodukten als ausgesprochen aufwendig dar.

Die trenntechnische Bearbeitung des Produktgemischs umfasst dabei
herkömmlicherweise eine Kühlung auf Umgebungstemperatur. Hierbei kondensiert der überwiegende Teil des in dem Produktgemisch enthaltenen Wassers aus. Die Kühlung erfolgt auf dem Druck, auf dem das Produktgemisch stromab der oxidativen Kopplung von Methan vorliegt, also typischerweise 5 bis 10 bar, beispielsweise 7 bis 8 bar. Anschließend erfolgen eine Verdichtung und anschließend eine Entfernung des Kohlendioxids, beispielsweise unter Verwendung einer Amin- und/oder Laugenwäsche, aus dem Produktgemisch. Restwasser wird anschließend typischerweise adsorptiv, beispielsweise unter Verwendung von Molsiebadsorbern, abgetrennt.

Ein durch eine entsprechende Aufbereitung aus dem Produktgemisch erhaltenes Gasgemisch wird einer Tieftemperaturtrennung zugeführt, in der Methan und tiefer als Methan siedende Komponenten, also insbesondere Kohlenmonoxid und Wasserstoff, sofern enthalten, zumindest überwiegend abgetrennt werden. Das restliche Kohlenwasserstoffgemisch enthält überwiegend oder ausschließlich Ethylen und höher als Ethylen siedende Verbindungen, soweit in dem Produktgemisch enthalten.

Verfahren zur Abtrennung von Methan und tiefer siedenden Verbindungen aus unterschiedlichen Kohlenwasserstoffgemischen, beispielsweise aus Erdgas oder aus Gasgemischen, die unter Verwendung von Dampfspaltverfahren erhalten wurden, sind aus dem Stand der Technik grundsätzlich bekannt. Diese Verfahren können jedoch bei der trenntechnischen Bearbeitung von Gasgemischen, die unter Einsatz der oxidativen Kopplung von Methan erhalten wurden, Nachteile aufweisen. Beispielsweise ist das für Erdgas verwendete sogenannte Recycle-Split-Vapor-(RSV-)Verfahren der Firma Ortloff, wie es in der US 4,157,904 A offenbart und bei H.-W. Häring (Hrsg.), Industrial Gases Processing, Wiley-VCH, 2006, Abschnitt 7.6.2, "Separation of Liquefied Petroleum Gas", näher erläutert ist, für die Trennung eines Gasgemischs, das unter Einsatz der oxidativen Kopplung von Methan erhalten wurde, nicht geeignet, da sich aufgrund der abweichenden Zusammensetzung die erforderlichen Reinheitennicht ohne weiteres erzielen ließen.

Die vorliegende Erfindung will daher insbesondere die Abtrennung von Methan und tiefer siedender Verbindungen aus einem Gasgemisch, das unter Einsatz der oxidativen Kopplung von Methan erhalten wurde, gegenüber dem Stand der Technik verbessern und energetisch und/oder apparativ optimieren.

### Offenbarung der Erfindung

Vor diesem Hintergrund schlägt die vorliegende Erfindung ein Verfahren zur Gewinnung eines oder mehrerer Olefine und eine entsprechende Anlage gemäß den Oberbegriffen der jeweiligen unabhängigen Patentansprüche vor. Ausgestaltungen sind jeweils Gegenstand der abhängigen Patentansprüche sowie der nachfolgenden Beschreibung.

Vor der Erläuterung der Merkmale und Vorteile der vorliegenden Erfindung werden noch einige Grundlagen und die verwendeten Begriffe erläutert.

Ist nachfolgend davon die Rede, dass ein Gasgemisch "unter Einsatz eines Verfahrens zur oxidativen Kopplung von Methan" gebildet wird, soll hierunter verstanden werden, dass bei der Bildung des Produktgemischs ein Verfahren beteiligt ist, das die zuvor erläuterten Reaktionen umfasst, insbesondere die Bildung von Methylradikalen, deren Kopplung zu Ethan und die anschließende Oxydehydrierung. Zusätzlich können jedoch weitere Verfahren bzw. Verfahrensschritte umfasst sein, insbesondere das oben erläuterte postkatalytische Dampfspalten. Diesem oder diesen weiteren Verfahren oder Verfahrensschritten können weitere Einsätze zugeführt werden, die zumindest teilweise zu Produkten umgesetzt werden, welche sich in dem Gasgemisch wiederfinden. Mit anderen Worten muss bei einer Bildung des Gasgemischs, die "unter Einsatz" des Verfahrens zur oxidativen Kopplung von Methan erfolgt, nicht nur die oxidative Kopplung von Methan beteiligt sein.

In entsprechender Weise soll nachfolgend unter einem "Reaktor, der zur oxidativen Kopplung von Methan eingerichtet ist" ein Reaktor verstanden werden, der zumindest in einer Reaktorzone einen Katalysator aufweist der sich für die oxidative Kopplung von Methan eignet. Zumindest in dieser Reaktorzone können durch geeignete Mittel, beispielsweise durch Brenner und vorgeschaltete Verdichter, Temperatur- und Druckbedingungen geschaffen werden, die unter Einfluss des Katalysators zu einer oxidativen Kopplung von Methan führen, wie zuvor erläutert. Ein entsprechender Reaktor kann neben der erläuterten Reaktorzone weitere Reaktorzonen aufweisen, insbesondere eine stromab der katalytischen Zone angeordnete nichtkatalytische Zone, die zum erläuterten postkatalytischen Dampfspalten verwendet wird, und in die weitere Kohlenwasserstoffe eingespeist werden können.

Flüssige und gasförmige Gemische können im hier verwendeten Sprachgebrauch reich oder arm an einer oder mehreren Komponenten sein, wobei "reich" für einen Gehalt von wenigstens 50%, 75%, 90%, 95%, 99%, 99,5%, 99,9% oder 99,99% und "arm" für einen Gehalt von höchstens 50%, 25%, 10%, 5%, 1%, 0,1% oder 0,01% auf molarer, Gewichts- oder Volumenbasis stehen kann. Der Begriff "überwiegend" kann der Definition von "reich" entsprechen.

Die vorliegende Anmeldung verwendet zur Charakterisierung von Drücken und Temperaturen die Begriffe "Druckniveau" und "Temperaturniveau", wodurch zum Ausdruck gebracht werden soll, dass entsprechende Drücke und Temperaturen in einer entsprechenden Anlage nicht in Form exakter Druck- bzw. Temperaturwerte verwendet werden müssen, um das erfinderische Konzept zu verwirklichen. Jedoch bewegen sich derartige Drücke und Temperaturen typischerweise in bestimmten Bereichen, deren Maximal- und Minimalwerte sich um beispielsweise nicht mehr als 1%, 5%, 10%, 20% oder sogar 50% unterscheiden.

Entsprechende Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste, beispielsweise aufgrund von Abkühlungseffekten, ein. Entsprechendes gilt für Temperaturniveaus. Bei den hier in bar angegebenen Druckniveaus handelt es sich jeweils um Absolutdrücke.

Bei einer "Rektifikationskolonne" handelt es sich im hier verwendeten Sprachgebrauch um eine Trenneinheit, die dafür eingerichtet ist, ein gasförmig oder flüssig oder in Form eines Zweiphasengemischs mit flüssigen und gasförmigen Anteilen, ggf. auch im überkritischen Zustand, eingespeistes Stoffgemisch durch Rektifikation zumindest teilweise aufzutrennen, also aus dem Stoffgemisch jeweils Reinstoffe oder zumindest Stoffgemische mit anderer Zusammensetzung zu erzeugen.

Typischerweise sind Rektifikationskolonnen als zylindrische Metallbehälter ausgebildet, die mit Einbauten, beispielsweise Siebböden oder geordneten oder ungeordneten Packungen, ausgerüstet sind. Neben den zur eigentlichen Trennung vorgesehenen Bereichen, die nachfolgend auch als "Trennbereiche" bezeichnet werden, können Rektifikationskolonnen auch für andere Zwecke vorgesehene Bereiche aufweisen, beispielsweise Kondensationsräume, die lediglich zur Phasentrennung von Zweiphasengemischen eingerichtet sind und auf diese Weise im Wesentlichen einen entsprechenden extern ausgebildeten Behälter ersetzen.

Eine Rektifikationskolonne weist einen Sumpfverdampfer auf. Hierbei handelt es sich um eine Einrichtung mit einem Wärmetauscher, der beheizt wird, und der dafür eingerichtet ist, eine im Sumpf der Rektifikationskolonne anfallende flüssige Fraktion, auch als Sumpfflüssigkeit bezeichnet, zu erwärmen. Mittels eines Sumpfverdampfers wird kontinuierlich ein Teil des Sumpfprodukts verdampft und gasförmig in dem Trennbereich zurückgespeist werden.

Im Gegensatz zu einer Rektifikationskolonne verfügt eine "Absorptionskolonne" nicht über einen Sumpfverdampfer, aber über eine kopfseitige Einspeisemöglichkeit für eine Absorptionsflüssigkeit. Absorptionskolonnen werden zur Absorption im Phasengegenstrom verwendet und daher auch als Gegenstromkolonnen bezeichnet. Bei der Absorption im Phasengegenstrom strömt die abgebende Gasphase aufwärts durch die Absorptionskolonne. Die aufnehmende Absorptionsflüssigkeit fließt, von oben aufgegeben und unten abgezogen, der Gasphase entgegen. In einer Absorptionskolonne sind ebenfalls typischerweise Einbauten vorgesehen, die für einen stufenweisen oder stetigen Phasenkontakt sorgen.

Im hier verwendeten Sprachgebrauch wird unter einem "Absorbat" die im Sumpf einer Absorptionskolonne anfallende, beladene Absorptionsflüssigkeit verstanden. Ein entsprechendes Absorbat enthält die flüssig den Sumpf der Absorptionskolonne erreichenden Komponenten der Absorptionsflüssigkeit und die aus der Gasphase absorbierten Komponenten. Die nicht absorbierten Komponenten gehen nicht in das Absorbat über. Die verwendete Definition entspricht jener gängiger Lehrbücher, beispielsweise Kapitel 10.1, "Absorption", in: B. Lohrengel, "Einführung in die thermischen Trennverfahren: Trennung von Gas-, Dampf- und Flüssigkeitsgemischen", 2. Auflage, De Gruyter, Oldenbourg, Berlin 2012.

Ein "Kondensatorverdampfer" weist einen Verflüssigungsraum und einen Verdampfungsraum auf. Verdampfungs- und Verflüssigungsraum werden typischerweise durch Gruppen von Passagen (Verflüssigungs- bzw. Verdampfungspassagen) eines Wärmetauschers gebildet, die untereinander in fluidischer Verbindung stehen. In dem Verflüssigungsraum wird die Kondensation eines ersten Fluidstroms durchgeführt, in dem Verdampfungsraum die Verdampfung eines zweiten Fluidstroms. Die beiden Fluidströme stehen dabei in indirektem Wärmetausch. Ein Kondensatorverdampfer kann in eine Rektifikationskolonne integriert sein. Kondensatorverdampfer können aber auch außerhalb einer entsprechenden Rektifikationskolonne angeordnet sein. In einem entsprechenden Kondensatorverdampfer einer Rektifikationskolonne wird aus einem Trennbereich gasförmig aufsteigendes Fluid, also das sogenannte "Kopfgas", zumindest teilweise kondensiert und kann als flüssiger Rücklauf auf den Trennbereich zurückgeführt werden. Ein entsprechender Kondensatorverdampfer wird dabei mit einem Fluid gekühlt, das unter den herrschenden Druckbedingungen die zumindest teilweise Kondensation des Kopfgases ermöglicht.

### Vorteile der Erfindung

Die vorliegende Erfindung betrifft die Trennung eines Gasgemischs, das unter Einsatz der oxidativen Kopplung von Methan bereitgestellt wird, und das Methan, tiefer als Methan siedende Komponenten und höher als Methan siedende Kohlenwasserstoffe aufweist. Dieses Gasgemisch kann insbesondere aus einem Produktgemisch einer oxidativen Kopplung von Methan gebildet werden, indem dieses verdichtet und danach zumindest größtenteils von Wasser und Kohlendioxid befreit wird. Bei der Bildung des Produktgemischs können, wie erwähnt, auch weitere Verfahren oder Verfahrensschritte wie das postkatalytische Dampfspalten beteiligt sein.

Wie insoweit bekannt, kann ein derartiges Gasgemisch in einer Rektifikationskolonne aufgetrennt werden, auf die ein Rücklauf aufgegeben wird, welcher durch Teilkondensation von Kopfgas der Rektifikationskolonne als Kondensat gebildet wird. Das Kopfgas enthält überwiegend oder ausschließlich Methan und tiefer als Methan siedende Verbindungen. Ein weiterer Teil des Kopfgases, insbesondere dessen bei der Teilkondensation gasförmig verbleibender Teil, kann stromab der Rektifikationskolonne einer oder mehreren weiteren Trenneinrichtungen zugeführt werden, beispielsweise um Methan abzutrennen, das in die oxidative Kopplung von Methan zurückgeführt werden soll.

Die vorliegende Erfindung beruht nun auf der Erkenntnis, dass es besonders vorteilhaft ist, wenn ein entsprechendes Gasgemisch einer Tieftemperaturrektifikation in einer Rektifikationskolonne mit zwei oder mehr Trennbereichen unterworfen wird,wobei die Trennbereiche fluidisch miteinander verbunden sind. Hierbei erfolgt eine Zwischenkondensation unter Verwendung eines oder mehrerer Kondensatorverdampfer, der oder die verfahrenstechnisch zwischen den Trennbereichen angeordnet sind.

Eine derartige Rektifikation kann, insbesondere gegenüber den eingangs erwähnten, herkömmlichen Verfahren bei deutlich geringeren Drücken erfolgen. Eine Anordnung des oder der Kondensatorverdampfer "verfahrenstechnisch zwischen" entsprechenden Trennbereichen bedeutet damit nicht oder zumindest nicht notwendigerweise eine räumliche Zwischenschaltung in dem Sinn, dass die jeweiligen Teile geodätisch übereinander und der jeweilge Kondensatorverdampfer geodätisch oberhalb des ersten und unterhalb des zweiten Trennbereichs angeordnet ist. Vielmehr ist hiermit gemeint, dass ein Kopfprodukt eines Trennbereichs unterhalb des obersten Trennbereichs kondensiert werden kann, der damit in einem Zwischenbereich der gesamten Rektifikation in der Gesamtsäule angeordnet ist.

Bei der Tieftemperaturrektifikation wird die Rektifikationskolonne in herkömmlichen Verfahren mit einem Rücklauf betrieben, der durch Kondensieren eines Teils des Kopfgases der Rektifikationskolonne gebildet wird. Reines Methan besitzt bei -97 °C einen Siededruck von ca. 29 bar. Ist neben Methan in einem entsprechenden Gasgemisch auch Wasserstoff, in dem geschilderten Gasgemisch beispielsweise in einem Gehalt von 5 bis 10 Molprozent, enthalten, erhöht sich der Siededruck deutlich.

Daher müsste, um die Kondensation des wasserstoffhaltigen Kopfgases zu ermöglichen, in herkömmlichen Verfahren entweder der Rektifikationsdruck erhöht oder auf tiefer siedende Kältemittel wie Methan zurückgegriffen werden. Eine Kondensation des Kopfgases bei einem Rektifikationsdruck von unter 30 bar und einer Verwendung von Ethylen als Kältemittel ist nicht möglich, weil sich Ethylen unter üblichen Bedingungen, d.h. als sogenanntes "Niederdruckethylen" auf einem Druck von knapp über 1 bar, nicht unter ca. -102 °C abküh It und damit ein Gasgemisch mittels Ethylen nur auf ca. -100 °C abgekühlt werde n kann.

Die hohen Rektifikationsdrücke gemäß der ersten Alternative erfordern einerseits kostenintensive, weil entsprechend hochdruckfeste, Rektifikationskolonnen. Andererseits ist die Rektifikation bei entsprechend hohen Drücken deutlich aufwendiger, weil sich die Dichtedifferenz zwischen Gas und Flüssigkeit verringert. Die relativen Flüchtigkeiten nähern sich bei entsprechend hohen Drücken aneinander an und die Trennung wird thermodynamisch schwieriger. Eine entsprechende Rektifikationskolonne muss daher auch ein deutlich größeres Volumen aufweisen. Dies erhöht neben der erforderlichen Druckfestigkeit ebenfalls die Baukosten einer entsprechenden Rektifikationskolonne. Bei größeren zu bearbeitenden Volumenströmen ist der Einsatz zweier oder mehrerer parallel angeordneter Rektifikationskolonnen erforderlich, um nicht an Baubarkeitsgrenzen zu stoßen. Dies verteuert entsprechende Anlagen weiter.

Eine Verwendung großer Mengen extern bereitgestellter, tiefer als Ethylen siedender Kältemittel gemäß der zweiten Alternative ist aufgrund des zusätzlichen Aufwands ihrer Bereitstellung ebenfalls in der Regel nicht erwünscht. Eine Möglichkeit, tiefere Temperaturen als mit Ethylen zu erreichen, besteht aber beispielsweise darin, das Kopfgas aus der Tieftemperaturrektifikation, das ja überwiegend oder ausschließlich Methan und Wasserstoff enthält, teilweise zu entspannen. Dies gilt insbesondere dann, wenn das Kopfgas auf einem geringeren Druckniveau als dem Druckniveau der Tieftemperaturrektifikation genutzt werden kann. Allerdings reicht die Kältemenge, die auf diese Weise gebildet werden kann, typischerweise nicht aus, um das Kopfgas in der gewünschten Menge zu kondensieren.

Die vorliegende Erfindung kann jedoch nicht nur in Fällen zum Einsatz kommen, in denen entsprechende Anteile von Kopfgas aus der Tieftemperaturrektifikation zur Erzeugung tiefer Temperaturen entspannt werden und die dabei freiwerdende Kältemenge nicht ausreicht. Anstelle von Kopfgas kann auch beispielsweise Methan aus einem Methankältekreislauf oder ein entsprechend tief siedendes gemischtes Kältemittel verwendet werden. Ein entsprechender Kältekreislauf kann dann durch den Einsatz der vorliegenden Erfindung bewusst kleiner dimensioniert werden, um Energie einzusparen und die Erstellungskosten zu reduzieren. Auch in diesem Fall reicht die Kältemenge des entspannten Methans zur Kondensation nicht aus.

Gemäß der vorliegenden Erfindung wird daher nun zusätzlich zu dem nicht ausreichenden, tiefer als Ethylen siedenden Kältemittel am Kopf der Rektifikationskolonne, beispielsweise dem entspannten Kopfgas, Ethylen als Kältemittel eingesetzt. Der Einsatz erfolgt dabei aber nicht am Kopf der Rektifikationskolonne insgesamt, sondern an einer oder mehreren Position unterhalb, an der die Ethylentemperatur von ca. -102 °C ausrei cht, um eine Kondensation zu bewirken und damit einen Rücklauf zu erzeugen.

Bekanntermaßen bildet sich in einer Rektifikationskolonne aufgrund der Beheizung im Sumpf und der Kühlung am Kopf ein Temperaturprofil aus, das von unten nach oben in Richtung niedrigerer Temperaturen verläuft. In einer vorliegend betrachteten Rektifikationskolonne kann die Temperatur im Sumpf beispielsweise bei ca. 0 °C und die Temperatur am Kopf bei unter -100 °C liegen. Es existiert daher eine Stelle, an der auch mit Ethylen bei ca. -102 °C eine Kondensation möglich ist.

Die Stelle, an der Kondensation mittels Ethylen erfolgt, kann nicht beliebig abgesenkt werden, weil unterhalb hiervon und oberhalb der Einspeisestelle des zu trennenden Gasgemischs in die Rektifikationskolonne noch eine ausreichende Anzahl an Böden vorhanden sein muss, um ausreichend Ethylen aus dem Gasgemisch auswaschen zu können. Die Bodenzahl kann in einem vorgegebenen Raum nicht beliebig erhöht werden, weil ein minimales Rücklaufverhältnis gewahrt bleiben muss.

Daher ist die Position oder sind die Positionen, an der eine oder mehrere solcher Kondensationen durchgeführt werden, auf die jeweiligen Säulendimensionen abzustimmen. Da dem Fachmann beispielsweise das Temperaturprofil innerhalb einer Rektifikationskolonne und die Mindestanzahl an erforderlichen Böden bekannt ist oder er entsprechende Parameter beispielsweise durch Simulation ermitteln kann, kann er diese Position oder Positionen aber ohne weiteres auswählen. Die zweckmäßigen Dimensionen der Kolonnenabschnitte und damit auch die zweckmäßige Position des oder der zwischengeschalteten Kondensatorverdampfer können daher durch fachmännisches Handeln bestimmt werden.

Da in einer Rektifikationskolonne typischerweise am Kopf die geringsten Temperaturen erreicht werden, ergibt sich durch die Anordnung eines entsprechenden Kondensatorverdampfers, der beispielsweise mit Ethylenkältemittel oder einem entsprechenden, tiefkalten Fluid, betrieben wird, in einem Zwischenbereich der Rektifikation eine deutlich größere Temperaturdifferenz zur dort herrschenden Kolonnentemperatur als im obersten Bereich der gesamten Rektifikationskolonne. Die Rektifikation kann daher trotz Anwesenheit von Wasserstoff bei geringerem Druck erfolgen. Weil ein entsprechender Kondensatorverdampfer im Rahmen der vorliegenden Erfindung verfahrenstechnisch zwischen zwei Trennbereichen vorgesehen ist, kann in einem ersten Trennbereich unterhalb des Kondensatorverdampfers, in den auch der Trenneinsatz eingespeist wird, eine Vortrennung vorgenommen werden, die im Wesentlichen in einer Abreicherung des Gasgemischs an einem Teil der höher als Methan siedenden Kohlenwasserstoffe besteht. In einem zweiten Trennbereich oberhalb des Kondensatorverdampfers, in dem geringere Trenntemperaturen herrschen, muss daher nur noch ein geringeres Fluidvolumen in Form dieser Kohlenwasserstoffe von den leichteren Komponenten abgetrennt werden. Daher sinkt der Kältemittelbedarf.

Die vorliegende Erfindung schlägt insgesamt ein Verfahren zur Gewinnung eines oder mehrerer Olefine vor, bei dem unter Einsatz einer oxidativen Kopplung von Methan ein Wasserstoff, Methan, Kohlenmonoxid und höher als methansidende Kohlenwasserstoffe enthaltendes Gasgemisch gebildet und einer Tieftemperaturtrennung unterworfen wird.

Erfindungsgemäß ist vorgesehen, dass die Tieftemperaturtrennung unter Verwendung einer Rektifikationskolonne mit einem ersten Trennbereich, einem oberhalb des ersten Trennbereichs angeordneten zweiten Trennbereich und einem, insbesondere im oben erläuterten Sinn verfahrenstechnisch zwischen den ersten Trennbereich und dem zweiten Trennbereich angeordneten Kondensatorverdampfer durchgeführt wird. Es sei darauf hingewiesen, dass, wie bereits erwähnt, im Rahmen der vorliegenden Erfindung auch mehrere Kondensatorverdampfer und dann auch zusätzliche Trennbereiche vorgesehen sein können. Die vorliegende Erfindung ist also nicht auf die Verwendung lediglich eines Kondensatorverdampfers und zweier Trennbereiche beschränkt.

Im Rahmen der vorliegenden Erfindung wird das Gasgemisch abgekühlt, zumindest teilweise als erster Trenneinsatz in den ersten Trennbereich eingespeist und in dem ersten Trennbereich unter Bildung eines ersten Kopfgases und einer ersten Sumpfflüssigkeit einer ersten Rektifikation unterworfen. Bei dieser ersten Rektifikation handelt es sich um die bereits zuvor erwähnte "Vortrennung" unter Verwendung des genannten Kondensatorverdampfers als Zwischenkondensator. Dieser Kondensatorverdampfer kann mit, wie erwähnt, Ethylenkältemittel bzw. einem entsprechenden Fluid auf ausreichend niedrigen Temperaturniveau betrieben werden, wie auch nachfolgend noch erläutert. Wird ein entsprechender Kondensatorverdampfer verfahrenstechnisch oberhalb des ersten Trennbereichs, nicht jedoch oberhalb der verwendeten Rektifikationskolonne insgesamt, also sämtlicher Trennbereiche oder eines einzigen Trennbereichs, angeordnet, ergibt sich hierdurch, wie erwähnt, ein größerer Temperaturabstand zur Trenntemperatur an der Stelle der Rektifikationskolonne, an der der Kondensatorverdampfer angeordnet ist, im Vergleich zu dem Temperaturabstand am Kopf der Rektifikationskolonne.

Im Rahmen der vorliegenden Erfindung wird unter Verwendung eines ersten Anteils des ersten Kopfgases in dem Kondensatorverdampfer ein Kondensat, das auf den ersten Trennbereich zurückgeführt wird, und unter Verwendung eines zweiten Anteils des Kopfgases ein zweiter Trenneinsatz, der in den zweiten Trennbereich eingespeist wird, gebildet. Das vorgetrennte Gasgemisch wird also einer weiteren Trennung in dem zweiten, oberen Trennbereich zugeführt. Dabei wird der zweite Trenneinsatz in dem zweiten Trennbereich unter Bildung eines zweiten Kopfgases und einer zweiten Sumpfflüssigkeit einer zweiten Rektifikation unterworfen. Diese zweite Rektifikation erfolgt, wie bei einer Rektifikation üblich, ebenfalls unter Verwendung eines flüssigen Rücklaufs, dessen Bereitstellung unten näher erläutert wird. Eine Flüssigkeit wird insbesondere aus einer unteren Region des zweiten Trennbereichs auf den ersten Trennbereich zurückgeführt. Wie erwähnt, sind die zu trennenden Volumina durch die bereits erfolgte Vortrennung in dem ersten Trennbereich in dem zweiten Trennbereich deutlich geringer als im Verfahren gemäß dem Stand der Technik. Eine entsprechende Rektifikationskolonne insgesamt kann daher bei deutlich geringeren Drücken betrieben werden, als sie im Stand der Technik verwendet werden.

Insbesondere können im Rahmen der vorliegenden Erfindung der erste Trennbereich und der zweite Trennbereich innerhalb einer gemeinsamen Außenhülle einer entsprechenden Rektifikationskolonne angeordnet sein. Auf diese Weise lässt sich eine integrierte Einheit schaffen, die insbesondere Isolationsvorteile bietet, beispielsweise in einer gemeinsamen Coldbox angeordnet werden kann. Der Kondensatorverdampfer kann dabei baulich zwischen, aber auch am Kopf einer entsprechenden Rektifikationskolonne angeordnet sein. Da der Betriebsdruck der Trennbereiche jedoch typischerweise höher als jener des Kondensatorverdampfers ist, wird dieser vorzugsweise außerhalb einer gemeinsamen (Druck-)Außenhülle angeordnet, die den ersten und zweiten Trennbereich ggf. umschließt.

Neben den genannten Trennbereichen und dem Kondensatorverdampfer umfasst eine Rektifikationskolonne, wie sie im Rahmen der vorliegenden Erfindung eingesetzt werden kann, insbesondere auch Leerräume, die hier auch als "Kondensationsräume" bezeichnet werden, und die dafür eingerichtet sind, mit Fluid beschickt zu werden, welches sich in entsprechenden Leerräumen in eine Gasphase und in eine flüssige Phase trennt. Entsprechende Leerräume dienen damit zur Phasentrennung entsprechend herkömmlicher Abscheidebehälter. Entsprechende Leerräume können insbesondere durch Flüssigkeitssperrböden von den Trennbereichen getrennt werden. Eine sich in einem entsprechenden Leerraum abscheidende Flüssigkeitsphase kann über einen Überlauf auf einen darunterliegenden Trennbereich abfließen.

Insbesondere sind im Rahmen der vorliegenden Erfindung die Trennbereiche innerhalb der Rektifikationskolonne durch eine flüssigkeits- und gasundurchlässige Trennwand voneinander getrennt, d. h. ein Gas- und Flüssigkeitsaustausch zwischen den Trennbereichen erfolgt lediglich durch hierfür gezielt angelegte Leitungen und kann daher kontrolliert durchgeführt werden.

Im Rahmen der vorliegenden Erfindung erfolgt das Abkühlen des Gasgemischs vor dem zumindest teilweisen Einspeisen desselben als ersten Trenneinsatz in den ersten Trennbereich auf ein Temperaturniveau von -70 bis -95 °C. Für diese Abkühlung kann ein auch unten noch näher erläuterter Wärmetauscher eingesetzt werden, der mit in der Rektifikation gebildeten Strömen gekühlt werden kann.

Vorteilhafterweise liegt ein Druckniveau, auf dem die Rektifikationskolonne betrieben wird, bei 24 bis 36 bar. Die Trennbereiche können dabei auf leicht unterschiedlichen Drücken betrieben werden, durch die sichergestellt werden kann, dass Fluide in der gewünschten Weise und insbesondere ohne zusätzliche Pumpen von dem ersten Trennbereich in den zweiten Trennbereich übergehen.

Vorteilhafterweise wird unter Verwendung eines ersten Anteils des zweiten Kopfgases, d. h. des entsprechenden Trennprodukts des zweiten, oberen Trennbereichs, ein Kondensat, das auf den zweiten Trennbereich zurückgeführt wird, gebildet, wobei ein zweiter Anteil des zweiten Kopfgases auf ein tieferes Druckniveau entspannt und anschließend einen Wärmetausch mit dem ersten Anteil des zweiten Kopfgases unterworfen wird. Auf diese Weise kann eine Kondensation des Kopfgases des zweiten, oberen Trennbereichs durchgeführt werden. Die Entspannung des zweiten Anteils des zweiten Kopfprodukts liefert dabei Kälte auf einem Temperaturniveau typischer sogenannter C1-Kälte. Diese ist dafür geeignet, ein entsprechendes zweites Kopfprodukt des zweiten Trennbereichs zumindest teilweise zu kondensieren, selbst wenn dieses nennenswerte Anteile an Wasserstoff enthält. Aufgrund der Zweiteilung der Rektifikationskolonne in dem ersten Trennbereich und dem zweiten Trennbereich und dem zwischengeschalteten Kondensatorverdampfer reicht die Expansionskälte, die bei der Entspannung des zweiten Anteils des zweiten Kopfgases gebildet wird, dabei zur Kondensation aus. Dies ist insbesondere darauf zurückzuführen, dass hier aufgrund der erfolgten Vortrennung, wie mehrfach erläutert, geringere Volumina kondensiert werden müssen.

Besonders vorteilhaft ist es, wenn der zweite Anteil des zweiten Kopfgases nach der Entspannung von dem auf das tiefere Druckniveau und dem Wärmetausch mit dem ersten Anteil des zweiten Kopfgases von dem tieferen Druckniveau auf ein höheres Druckniveau verdichtet wird. Für diese Verdichtung kann insbesondere, wie nachfolgend erläutert, eine Anordnung aus einer Expansionsturbine, mit der die vorige Entspannung des zweiten Anteils des zweiten Kopfgases durchgeführt wird, und einem Turboverdichter, in dem die Verdichtung erfolgt, durchgeführt werden. Auf diese Weise kann bei der Entspannung freiwerdende Arbeit zurückgewonnen und ein entsprechendes Kopfprodukt zugleich auf einen gewünschten Druck verdichtet werden. Mit anderen Worten wird also zum Entspannen des zweiten Anteils des zweiten Kopfgases von dem auf das tiefere Druckniveau im Rahmen der vorliegenden Erfindung vorteilhafterweise ein Turboexpander und zum Verdichten des zweiten Kopfgases von dem tieferen Druckniveau auf das höhere Druckniveau vorteilhafterweise ein durch den Turboexpander angetriebener Turboverdichter verwendet.

Vorteilhafterweise liegt im Rahmen der vorliegenden Erfindung das tiefere Druckniveau bei 6 bis 11 bar und das höhere Druckniveau bei 10 bis bis 15 bar. Dies ermöglicht es, die bereits erwähnte Kältemenge zur Kondensation des ersten Anteils des zweiten Kopfprodukts bereitzustellen.

Im Rahmen der vorliegenden Erfindung ist vorteilhafterweise vorgesehen, das der zweite Anteil des zweiten Kopfgases nach der Entspannung auf das tiefere Druckniveau, nach dem Wärmetausch mit dem ersten Anteils des zweiten Kopfgases, und vor dem Verdichten von dem tieferen Druckniveau auf das höhere Druckniveau einem Wärmetausch mit dem Gasgemisch, das zumindest teilweise als erster Trenneinsatz in den ersten Trenneinsatz eingespeist wird, zu dessen Abkühlung unterworfen wird. Auf diese Weise kann auch nach der Verwendung des ersten Anteils des zweiten Kopfgases zur zumindest teilweisen Kondensation des ersten Anteils des zweiten Kopfgases verbliebene Kälte zur Abkühlung des ersten Trenneinsatzes verwendet werden.

Wie bereits erwähnt, kann der Kondensatorverdampfer im Rahmen der vorliegenden Erfindung vorteilhafterweise mittels Ethylenkältemittel oder mittels eines Fluids auf einem entsprechenden Temperaturniveau gekühlt werden. Der Einsatz von Ethylenkältemittel aus einem Ethylenkältekreislauf ist dabei grundsätzlich bekannt. Ethylenkältemittel kann insbesondere auch unter Verwendung von in einem entsprechenden Verfahren gebildeten Fluiden gebildet werden. Ethylenkältemittel kann auch an anderer Stelle als in dem Kondensatorverdampfer eingesetzt werden.

Besonders vorteilhaft kann es jedoch auch sein, wenn der Kondensatorverdampfer unter Verwendung zumindest eines Teils der ersten Sumpfflüssigkeit gekühlt wird, also der bei der ersten Rektifikation anfallenden Sumpfflüssigkeit. Diese weist überwiegend höher als Methan siedende Kohlenwasserstoffe auf. Hierbei handelt es sich überwiegend um Kohlenwasserstoffe mit zwei Kohlenstoffatomen, also Ethan und Ethylen. Es kann daher ausreichend sein, eine entsprechende Sumpfflüssigkeit als Kältemittel in einem entsprechenden Kondensatorverdampfer zu verwenden. Dies trifft insbesondere dann zu, wenn ein entsprechendes Fluid im gasförmigen Zustand bereitgestellt werden soll und daher in dem Kondensatorverdampfer zumindest teilweise verdampft werden kann.

Im Rahmen der vorliegenden Erfindung umfasst die erste Sumpfflüssigkeit, wie erwähnt, insbesondere höher als Methan siedende Kohlenwasserstoffe, die zuvor in dem ersten Trenneinsatz bzw. dem Gasgemisch enthalten waren. Das zweite Kopfgas umfasst hingegen überwiegend oder ausschließlich Wasserstoff, Methan und Kohlenmonoxid, die aus dem Gasgemisch bzw. dem ersten Trenneinsatz stammen und über den zweiten Trenneinsatz in den zweiten Trennbereich übergegangen sind. Das erste Kopfgas bzw. der zweite Trenneinsatz sind gegenüber dem ersten Trenneinsatz an entsprechenden leichten Komponenten angereichert und an schwereren Komponenten abgereichert.

Die vorliegende Erfindung betrifft auch eine Anlage zur Gewinnung eines oder mehrerer Olefine, mit Mitteln, die dafür eingerichtet sind, unter Einsatz einer oxidativen Kopplung von Methan eine Wasserstoff, Methan, Kohlenmonoxid und höher als methansiedende Kohlenwasserstoff enthaltendes Gasgemisch zu bilden und einer Tieftemperaturtrennung zu unterwerfen. Erfindungsgemäß ist vorgesehen, dass zur Tieftemperaturtrennung eine Rektifikationskolonne mit einem ersten Trennbereich, einem oberhalb des ersten Trennbereichs angeordneten zweiten Trennbereich und einem, insbesondere zwischen dem ersten Trennbereich und dem zweiten Trennbereich angeordneten Kondensatorverdampfer bereitgestellt ist. Ferner sind erfindungsgemäß Mittel vorgesehen, die dafür eingerichtet sind, das Gasgemisch abzukühlen, zumindest teilweise als ersten Trenneinsatz in den ersten Trennbereich einzuspeisen und in dem ersten Trennbereich unter Bildung eines ersten Kopfgases und einer ersten Sumpfflüssigkeit einer ersten Rektifikation zu unterwerfen. Diese Mittel sind ferner dafür vorgesehen, unter Verwendung eines ersten Anteils des ersten Kopfgases in dem Kondensatorverdampfer ein Kondensat zu bilden und auf den ersten Trennbereich zurückzuführen, unter Verwendung eines zweiten Anteils des ersten Kopfgases einen zweiten Trenneinsatz zu bilden und in den zweiten Trennbereich einzuspeisen, und den zweiten Trenneinsatz in dem zweiten Trennbereich unter Bildung eines zweiten Kopfgases und einer zweiten Sumpfflüssigkeit einer zweiten Rektifikation zu unterwerfen. Wie bereits erwähnt, können in einer entsprechenden Anlage vorteilhafterweise der erste Trennbereich und der zweite Trennbereich in einer gemeinsamen Außenhülle angeordnet sein.

Zu Merkmalen und Vorteilen einer entsprechenden Anlage sei auf die zuvor erläuterten Verfahrensmerkmale ausdrücklich verwiesen. Dies gilt auch für die vorteilhafterweise vorgesehene Anlage, die zusätzlich zur Durchführung eines Verfahrens eingerichtet ist, wie es zuvor erläutert wurde, und die hierzu eingerichtete Mittel aufweist.

Die Erfindung wird in bevorzugten Ausgestaltungen unter Bezugnahme auf die beigefügten Zeichnungen näher erläutert.

### Kurze Beschreibung der Zeichnung

Figur 1 veranschaulicht ein Verfahren gemäß einer Ausführungsform der Erfindung in Form eines vereinfachten Prozessflussdiagramms.
Figur 2 veranschaulicht eine Alternative des Verfahrens gemäß Figur 1 in Form eines vereinfachten Prozessflussdiagramms in Teilansicht.

### Ausführliche Beschreibung der Zeichnung

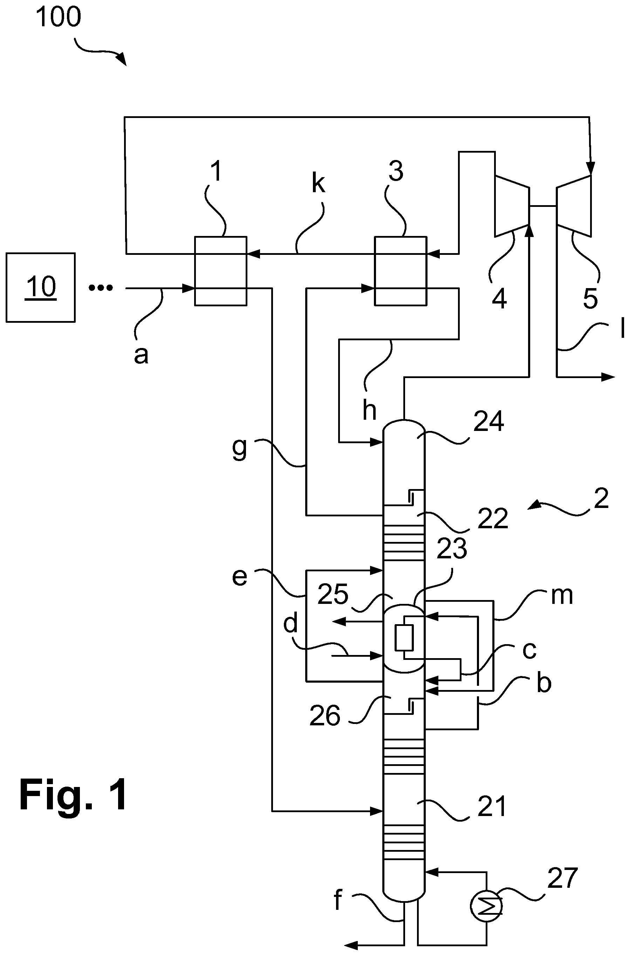

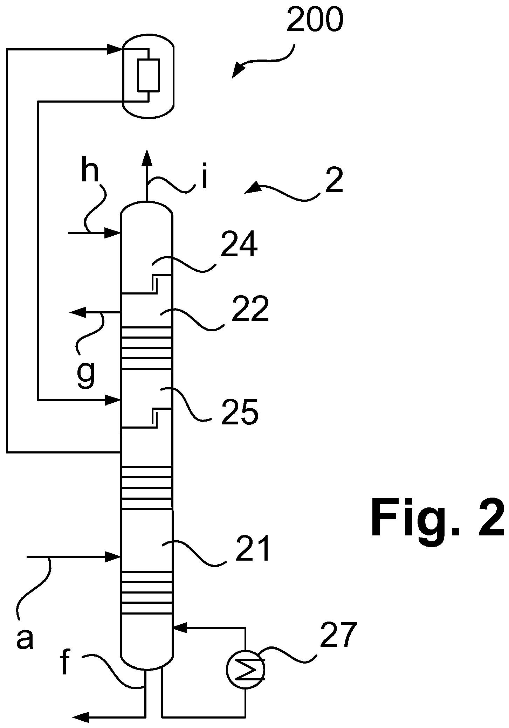

Figuren 1 und 2 veranschaulichen Verfahren gemäß Ausführungsformen der Erfindung in Form stark vereinfachter Prozessflussdiagramme, wobei in Figur 2 eine Teilansicht eines zu dem Verfahren gemäß Figur 1 alternativen Verfahrens dargestellt ist, in dem jedoch die übrigen, in Figur 1 gezeigten Komponenten verwendet werden. Die Verfahren sind insgesamt mit 100 bzw. 200 bezeichnet.

Die zu Figuren 1 und 2 erläuterten Komponenten bzw. Verfahrensschritte sind gleichzeitig Teil entsprechender Anlage bzw. dort vorrichtungsmäßig implementiert, so dass die nachfolgenden Erläuterungen auch entsprechende Anlagen betreffen. Die Darstellung ist insbesondere dahingehend stark vereinfacht, dass in der Praxis vorgesehene Vorrichtungen wie Regler, Ventile, für das Anfahren einer entsprechenden Anlage erforderliche Bypässe usw. nicht veranschaulicht sind. Der Fachmann sieht entsprechende Elemente nach Bedarf vor.

In den Verfahren 100 und 200 wird eine oxidative Kopplung von Methan eingesetzt, wie hier in sehr vereinfachter Form gezeigt und in Figur 1 mit 10 zusammengefasst. Der oxidativen Kopplung von Methan 10 können weitere Verfahren bzw. Verfahrensschritte zu- bzw. nachgeordnet sein, wie einleitend erläutert und hier durch Auslassungspunkte symbolisiert. Die oxidativen Kopplung von Methan 10 kann insbesondere mit einem oder mehreren methanreichen Stoffströmen gespeist werden, wie hier nicht dargestellt. Der oxidativen Kopplung von Methan 10 können ferner ein oder mehrere sauerstoffreiche Stoffströmen zugeführt werden, wie hier ebenfalls nicht dargestellt. In die oxidative Kopplung von Methan 1 kann auch insbesondere ein in den Verfahren 100 und 200 bzw. stromab hiervon gebildeter methanreicher Stoffstrom zurückgeführt werden. Wird ein eingangs erläutertes postkatalytisches Dampfspalten eingesetzt, können diesem ein oder mehrere paraffinreiche Stoffströme, beispielsweise ein oder mehrerer Ethan- oder Propanströme, zugeführt werden. Auch diese können in den Verfahren 100 und 200 oder stromab hiervon gebildet werden, wie auch nachfolgend noch näher erläutert.

Unter Einsatz der oxidativen Kopplung von Methan 10, d.h. auch ggf. unter Einsatz weiterer Verfahren bzw. Verfahrensschritte, insbesondere zur Aufbereitung eines Produktgemischs der oxidativen Kopplung von Methan 10 und ggf. des postkatalytischen Dampfspaltens, wird ein Gasgemisch bereitgestellt, das neben Methan tiefer als Methan siedende Komponenten, insbesondere Wasserstoff und Kohlenmonoxid, sowie höher als Methan siedende Kohlenwasserstoffe enthält, aber kein oder kaum Kohlendioxid und Wasser mehr. Kohlendioxid und Wasser wurden durch geeignete Trenneinrichtungen stromauf abgetrennt.

Ein entsprechendes Gasgemisch wird, wie hier in Form eines Stoffstroms a veranschaulicht, zunächst durch einen Wärmetauscher 1 geführt und in diesem abgekühlt. Das entsprechend abgekühlte Gasgemisch des Stoffstroms a wird in Form eines Trenneinsatzes, der hier als "erster" Trenneinsatz bezeichnet wird, in eine Rektifikationskolonne 2 eingespeist.

Die Rektifikationskolonne 2 umfasst in den dargestellten Ausführungsformen einen ersten Trennbereich 21 und einen zweiten Trennbereich 22. Entsprechende Trennbereiche sind insbesondere durch Trennböden untergliedert, wohingegen andere Bereiche, nämlich Leerräume 24, 25 und 26 nicht durch entsprechende Trennböden unterteilt sind. Neben den Trennbereichen 21 und 22 und den Leerräumen 24, 25 und 26 ist ein Kondensatorverdampfer 23 vorgesehen, der hier auch baulich zwischen den übereinander angeordneten Trennbereichen 21 und 22 angeordnet ist. Eine alternative Anordnung ist in Figur 2 veranschaulicht.

In dem ersten Trennbereich 21 der Rektifikationskolonne 2 wird der erste Trenneinsatz, d. h. das in Form des Stoffstroms a eingespeiste Fluid, einer Rektifikation unterworfen, die hier auch als "erste" Rektifikation bezeichnet wird. Es werden eine Sumpfflüssigkeit und ein Kopfgas gebildet, die hier als "erste" Sumpfflüssigkeit und "erstes" Kopfgas bezeichnet werden. Das erste Kopfgas kann teilweise in Form eines Stoffstroms b gasförmig abgezogen, in dem Kondensatorverdampfer 23 zumindest zum Teil kondensiert und als Kondensat in Form eines Kondensatstroms c in den Leerraum 26 eingespeist werden. Ein sich in dem Leerraum 26 sammelndes Kondensat kann über einen Überlauf auf den ersten Trennbereich zurückgeführt werden. Ein zweiter Anteil des ersten Kopfgases kann in Form eines Stoffstroms e aus dem Leerraum 26 ausgeführt und teilweise oder vollständig in einen unteren Bereich des zweiten Trennbereichs 22 eingespeist werden. Aus einem unteren Bereich des zweiten Trennbereichs 22 wird ein Kondensat in Form eines Stoffstroms m in den Leerraum 26 überführt und kann, wie das Kondensat aus dem Kondensatorverdampfer 23, über den Überlauf auf den ersten Trennbereich 21 abfließen.

In dem zweiten Trennbereich 22 der Rektifikationskolonne 2 wird eine Rektifikation durchgeführt, die hier auch als "zweite" Rektifikation bezeichnet wird. Bei dieser wird ein Kopfgas gebildet, das hier auch als "zweites" Kopfgas bezeichnet wird. Dieses kann zu einem ersten Anteil in Form eines Stoffstroms g abgezogen, in einem Wärmetauscher 3 zumindest teilweise verflüssigt bzw. kondensiert und in Form eines Stoffstroms h in einen Leerraum 24 die Rektifikationskolonne 2 zurückgeführt werden. Sich in dem Leerraum 24 sammelndes Kondensat kann über einen Überlauf auf den zweiten Trennbereich 22 zurückgeführt werden.

Ein zweiter Anteil des zweiten Kopfgases, d. h. des Kopfgases des zweiten Trennbereichs 22, kann gasförmig aus dem Leerraum 24 abgezogen und in Form eines Stoffstroms i einer Entspannung in einer Expansionsturbine 4 zugeführt werden. Durch die Entspannung in der Expansionsturbine 4 verringert sich die Temperatur des Stoffstroms i, der nun mit k veranschaulicht ist. Der Stoffstrom i bzw. k kann in dem Wärmetauscher 3 einem Wärmetauscher mit dem ersten Anteil des zweiten Kopfgases des zweiten Trennbereichs 22, der in Form des Stoffstroms g durch den Wärmetauscher 3 geführt wird, unterworfen werden. Auf diese Weise ermöglicht die vorliegende Erfindung eine Kondensation des entsprechenden Kopfgasanteils. Nach dem Wärmetausch in dem Wärmetauscher 3 kann ein entsprechender Stoffstrom k durch den Wärmetauscher 1 geführt werden und hier durch den Stoffstrom a abkühlen. Anschließend kann der Stoffstrom k in einem Turboverdichter 5 der durch den Turboexpander 4 angetrieben werden kann, rückverdichtet und in Form eines Stoffstroms I aus dem Verfahren ausgeführt werden.

Die Rektifikationskolonne 2 kann unter Verwendung eines Sumpfverdampfers 27 betrieben werden. Die Sumpfflüssigkeit aus dem ersten Trennbereich 21 kann zumindest zum Teil in Form eines Stoffstroms f abgezogen werden, der, wie erwähnt, im Wesentlichen Kohlenwasserstoffe mit zwei und mehr Kohlenstoffatomen umfasst.

In dem in Figur 1 veranschaulichten Verfahren 100 wird der Kondensatorverdampfer 23 unter Verwendung eines Ethylenkältemittelstroms d gekühlt, der in einem Verdampfungsraum des Kondensatorverdampfers 23 verdampft wird. Der Kondensatorverdampfer 23 kann jedoch auch, wie hier nicht veranschaulicht, unter Verwendung des Stoffstroms f, also der ersten Sumpfflüssigkeit aus dem ersten Trennbereich 21 der Rektifikationskolonne 2, gekühlt werden. Auch dieser Stoffstrom f wird dabei verdampft und in verdampfter Form aus dem Verfahren ausgeführt.

Figur 2 veranschaulicht eine Alternative des Verfahrens gemäß Figur 1 in Form eines vereinfachten Prozessflussdiagramms in Teilansicht. Es ist nur die Rektifikationskolonne 2 gezeigt. Ihre Einbindung in das Verfahren 200 ergibt sich aus der Bezeichnung der Fluidströme. Diese Alternative entspricht einer besonders vorteilhaften Ausgestaltung des erfindungsgemäßen Verfahrens, weil hier die Trennbereiche 21 und 22, die auf einem vergleichbaren Druckniveau betrieben werden können, innerhalb einer gemeinsamen Außenhülle angeordnet werden können, der Kondensatorverdampfer 23, in dem beispielsweise Niederdruck-Ethylenkältemittel verwendet wird, jedoch nicht. Hierdurch kann für den Kondensatorverdampfer 23 auch beispielsweise ein geringerer Behälterdurchmesser verwendet werden. Der Kondensatorverdampfer kann aber auch im freien Volumen des zweiten Trennbereichs 22 angeordnet werden.

Der Ablauf von Flüssigkeit von dem zweiten Trennbereich 22 auf den ersten Trennbereich wird hier über einen Überlauf bewerkstelligt werden. Alternativ dazu ist aber auch eine Führung analog dem Strom m gemäß Figur 1 möglich.

## Patentansprüche

1. Verfahren (100, 200) zur Gewinnung eines oder mehrerer Olefine, bei dem unter Einsatz einer oxidativen Kopplung von Methan (10) ein Wasserstoff, Methan, Kohlenmonoxid und höher als Methan siedende Kohlenwasserstoffe enthaltendes Gasgemisch gebildet und einer Tieftemperaturtrennung (1-5) unterworfen wird, **dadurch gekennzeichnet, dass** die Tieftemperaturtrennung (1-5) unter Verwendung einer Rektifikationskolonne (2) mit einem ersten Trennbereich (21), einem oberhalb des ersten Trennbereichs (21) angeordneten zweiten Trennbereich (22) und einem Kondensatorverdampfer (23) durchgeführt wird, wobei wobei das Gasgemisch abgekühlt, zumindest teilweise als erster Trenneinsatz in den ersten Trennbereich (21) eingespeist und in dem ersten Trennbereich (21) unter Bildung eines ersten Kopfgases und einer ersten Sumpfflüssigkeit einer ersten Rektifikation unterworfen wird, wobei unter Verwendung eines ersten Anteils des ersten Kopfgases in dem Kondensatorverdampfer (23) ein Kondensat, das auf den ersten Trennbereich zurückgeführt wird, und unter Verwendung eines zweiten Anteils des Kopfgases ein zweiter Trenneinsatz, der in den zweiten Trennbereich (22) eingespeist wird, gebildet werden, und wobei der zweite Trenneinsatz in dem zweiten Trennbereich (22) unter Bildung eines zweiten Kopfgases und einer zweiten Sumpfflüssigkeit einer zweiten Rektifikation unterworfen wird.

2. Verfahren (100, 200) nach Anspruch 1, bei dem das Abkühlen des Gasgemischs vor dem zumindest teilweisen Einspeisen als erster Trenneinsatz in den ersten Trennbereich (21) auf ein Temperaturniveau von -70 bis -95 °C erfolgt.

3. Verfahren (100, 200) nach Anspruch 1 oder 2, bei dem das ein Druckniveau, auf dem die Rektifikationskolonne (2) betrieben wird, bei 24 bis 36 bar liegt.

4. Verfahren (100, 200) nach einem der vorstehenden Ansprüche, bei dem unter Verwendung eines ersten Anteils des zweiten Kopfgases ein Kondensat, das auf den ersten Trennbereich zurückgeführt wird, gebildet wird, wobei ein zweiter Anteil des zweiten Kopfgases auf ein tieferes Druckniveau entspannt und einem Wärmetausch mit dem ersten Anteil des zweiten Kopfgases unterworfen wird.

5. Verfahren (100, 200) nach Anspruch 4, bei dem der zweite Anteil des zweiten Kopfgases nach der Entspannung auf das tiefere Druckniveau und dem Wärmetausch mit dem ersten Anteil des zweiten Kopfgases von dem tieferen Druckniveau auf ein höheres Druckniveau verdichtet wird.

6. Verfahren (100, 200) nach Anspruch 5, bei dem zum Entspannen des zweiten Anteils des zweiten Kopfgases auf das tiefere Druckniveau ein Turboexpander (4) und zum Verdichten des zweiten Kopfgases von dem tieferen Druckniveau auf das höhere Druckniveau ein durch den Turboexpander (4) angetriebener Turboverdichter (5) verwendet wird.

7. Verfahren nach Anspruch 5 oder 6, bei dem das tiefere Druckniveau bei 6 bis 11 bar und das höhere Druckniveau bei 10 bis bis 15 bar liegt.

8. Verfahren nach einem der Ansprüche 5 bis 7, bei dem der zweite Anteil des zweiten Kopfgases nach der Entspannung auf das tiefere Druckniveau, nach dem Wärmetausch mit dem ersten Anteil des zweiten Kopfgases, und vor dem Verdichten von dem tieferen Druckniveau auf das höhere Druckniveau einem Wärmetausch mit dem Gasgemisch, das zumindest teilweise als erster Trenneinsatz in den ersten Trennbereich (21) eingespeist wird, zu dessen Abkühlung unterworfen wird.

9. Verfahren nach einem der vorstehenden Ansprüche, bei dem der Kondensatorverdampfer (23) mittels Ethylenkältemittel gekühlt wird.

10. Verfahren nach einem der vorstehenden Ansprüche, bei dem der Kondensatorverdampfer (23) unter Verwendung zumindest eines Teils der ersten Sumpfflüssigkeit gekühlt wird.

11. Anlage zur Gewinnung eines oder mehrerer Olefine, mit Mitteln, die dafür eingerichtet sind, unter Einsatz einer oxidativen Kopplung von Methan (10) ein Wasserstoff, Methan, Kohlenmonoxid und höher als Methan siedende Kohlenwasserstoffe enthaltendes Gasgemisch zu bilden und einer Tieftemperaturtrennung (1-5) zu unterwerfen, **dadurch gekennzeichnet, dass** zur Tieftemperaturtrennung (1-5) eine Rektifikationskolonne (2) mit einem ersten Trennbereich (21), einem oberhalb des ersten Trennbereichs (21) angeordneten zweiten Trennbereich (22) und einem Kondensatorverdampfer (23) vorgesehen ist, wobei Mittel vorgesehen sind, die dafür eingerichtet sind, das Gasgemisch abzukühlen, zumindest teilweise als ersten Trenneinsatz in den ersten Trennbereich (21) einzuspeisen und in dem ersten Trennbereich (21) unter Bildung eines ersten Kopfgases und einer ersten Sumpfflüssigkeit einer ersten Rektifikation zu unterwerfen, unter Verwendung eines ersten Anteils des ersten Kopfgases in dem Kondensatorverdampfer (23) ein Kondensat zu bilden und auf den ersten Trennbereich zurückzuführen, unter Verwendung eines zweiten Anteils des ersten Kopfgases einen zweiten Trenneinsatz zu bilden und in den zweiten Trennbereich (22) einzuspeisen, und den zweiten Trenneinsatz in dem zweiten Trennbereich unter Bildung eines zweiten Kopfgases und einer zweiten Sumpfflüssigkeit einer zweiten Rektifikation zu unterwerfen.

12. Anlage nach Anspruch 11, bei dem der erste Trennbereich (21), der zweite Trennbereich (22) und der Kondensatorverdampfer (23) in einer gemeinsamen Außenhülle angeordnet sind.

13. Anlage nach Anspruch 11 oder 12, die zur Durchführung eines Verfahrens nach einem der Ansprüche 1 bis 10 eingerichtet ist.
